(19) Europäisches Patentamt
European Patent Office
Office européen des brevets

(11) EP 2 844 139 B1

(12) FASCICULE DE BREVET EUROPEEN

(45) Date de publication et mention de la délivrance du brevet:
**29.06.2016 Bulletin 2016/26**

(21) Numéro de dépôt: **13722352.5**

(22) Date de dépôt: **03.05.2013**

(51) Int Cl.:
***A61B 5/04*** (2006.01) ***A61B 5/048*** (2006.01)
***A61M 16/00*** (2006.01) ***A61B 5/00*** (2006.01)
***G06F 19/00*** (2011.01)

(86) Numéro de dépôt international:
**PCT/EP2013/059279**

(87) Numéro de publication internationale:
**WO 2013/164462 (07.11.2013 Gazette 2013/45)**

# (54) PROCÉDÉ DE CARACTÉRISATION DE L'ÉTAT PHYSIOLOGIQUE D'UN PATIENT À PARTIR DE L'ANALYSE DE SON ACTIVITÉ ELECTRIQUE CÉREBRALE, ET DISPOSITIF DE SURVEILLANCE FAISANT APPLICATION

VERFAHREN ZUR CHARAKTERISIERUNG DES PHYSIOLOGISCHEN ZUSTANDS EINES PATIENTEN AUS DER ANALYSE DER ZEREBRALEN ELEKTRISCHEN AKTIVITÄT DES PATIENTEN SOWIE ÜBERWACHUNGSVORRICHTUNG ZUR ANWENDUNG DIESES VERFAHRENS

METHOD FOR CHARACTERISING THE PHYSIOLOGICAL STATE OF A PATIENT FROM THE ANALYSIS OF THE CEREBRAL ELECTRICAL ACTIVITY OF SAID PATIENT, AND MONITORING DEVICE APPLYING SAID METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.05.2012 FR 1254089**

(43) Date de publication de la demande:
**11.03.2015 Bulletin 2015/11**

(73) Titulaires:
- **Université Pierre et Marie Curie (Paris 6)**
  **75005 Paris (FR)**
- **Centre National de la Recherche Scientifique**
  **75016 Paris (FR)**
- **Institut du Cerveau et de la Moelle Épinière-ICM**
  **75013 Paris (FR)**
- **Institut National de la Santé et de la Recherche Médicale**
  **75654 Paris 13 (FR)**
- **Assistance Publique Hôpitaux De Paris**
  **75004 Paris 4 (FR)**

(72) Inventeurs:
- **SIMILOWSKI, Thomas**
  **92130 Issy-les-Moulineaux (FR)**
- **RAUX, Mathieu**
  **75013 Paris (FR)**
- **CHAVEZ, Mario**
  **94600 Choisy Le Roi (FR)**
- **MARTINERIE, Jacques**
  **91120 Palaiseau (FR)**
- **POUGET, Pierre**
  **75015 Paris (FR)**

(74) Mandataire: **Parzy, Benjamin Alain et al**
**Cabinet Boettcher**
**16, rue Médéric**
**75017 Paris (FR)**

(56) Documents cités:
**WO-A1-97/16216** **WO-A1-2010/060153**
**FR-A1- 2 903 314** **FR-A1- 2 962 322**
**US-A1- 2004 254 493** **US-A1- 2007 208 269**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention est relative à un procédé de caractérisation de l'état physiologique d'un patient à partir de l'analyse de son activité électrique cérébrale. L'invention est également relative à un dispositif de surveillance faisant application.

Bien qu'ayant été plus particulièrement mis au point à l'occasion d'un programme de recherche portant sur l'étude des dysharmonies pouvant intervenir entre un patient et le dispositif d'assistance respiratoire auquel le patient est relié, le procédé de l'invention a un champ d'application allant bien au-delà de cette application particulière.

En effet, comme le procédé de l'invention permet la caractérisation d'états physiologiques et donc la détection d'un état physiologique déviant d'un état physiologique de référence, il peut donc notamment servir à détecter des périodes cognitives spécifiques de certains états psychophysiologiques (niveaux de vigilance, reconnaissance visuelle) ou émotionnels (peur, joie...), ou encore à détecter des périodes pathologiques, comme par exemple une crise d'épilepsie en préparation, et fournir dans le cas échéant un signal d'alerte nécessaire pour permettre une prévention ou une intervention thérapeutique. Le procédé de l'invention peut encore servir, lors d'un sommeil, à différencier les différents stades de sommeil, ou, lors d'une anesthésie, à caractériser des stades d'endormissement sous anesthésie avec éventuellement un contrôle automatique de la régulation de la substance injectée.

ARRIERE PLAN DE L'INVENTION

L'assistance respiratoire mécanique constitue une mesure de suppléance vitale qui a pour objectif premier de pallier les défaillances aiguës ou chroniques de l'appareil respiratoire, comme par exemple au cours d'une pneumonie sévère. Elle consiste en la fourniture d'un volume prédéterminé de gaz ou en une pressurisation des voies aériennes, au moyen d'une interface (masque, sonde d'intubation ou canule de trachéotomie). Dans les deux cas, divers réglages permettent d'adapter le flux de gaz aux besoins du patient.

C'est une approche thérapeutique très fréquemment mise en oeuvre au bloc opératoire, en réanimation, en salle de surveillance post-interventionnelle, aux urgences voire dans les ambulances des Services Mobiles d'Urgence et de Réanimation (SMUR), ou encore à domicile, en cas d'insuffisance respiratoire. Elle concerne environ un patient sur deux admis en réanimation en France et a une incidence de 2,7 pour mille habitants aux Etats Unis, soit environ 250.000 patients par an en 2000 et une projection de 650.000 patients par an en 2020. Un tiers de ces patients sont ventilés plus de quatre jours.

Outre ses effets physiologiques d'assistance respiratoire, la ventilation mécanique soulage le symptôme dominant de la défaillance respiratoire qu'est la dyspnée. Elle doit ainsi remplir la double fonction d'améliorer l'oxygénation et l'épuration du dioxyde de carbone, et d'assurer le confort du patient. Faute de quoi, comme toute thérapeutique mal adaptée, elle peut devenir délétère et être responsable de complications iatrogéniques. Il est donc primordial que l'aide délivrée par la ventilation mécanique soit parfaitement adaptée aux besoins du patient. Ce dernier doit être en harmonie avec le ventilateur qui délivre la ventilation mécanique.

Autant les modalités de surveillance des effets physiologiques de l'assistance respiratoire sont bien codifiées et d'usage simple (mesures de grandeurs physiques dont les valeurs apparaissent sur l'écran du ventilateur), autant les modalités d'évaluation de l'harmonie patient ventilateur, considérée sous un angle sensoriel sont peu nombreuses, peu codifiées, et peu utilisées. C'est particulièrement vrai chez les patients pauci-communicants, situation fréquente en réanimation. L'enjeu est de détecter les situations au cours desquelles patient et ventilateur ne sont plus en harmonie. On parle alors de dysharmonie.

Cette dysharmonie n'est pas seulement inconfortable pour le patient. Elle apparaît délétère pour son devenir. En effet, la délivrance d'une assistance respiratoire non appropriée aux besoins du patient est associée à une augmentation de la durée de la ventilation mécanique et de séjour en réanimation, ainsi qu'à des troubles psychologiques de type syndrome de stress post-traumatique.

L'expérience quotidienne montre que la recherche d'une dysharmonie au moyen de l'examen clinique est difficile et peu pratiquée. Elle impose en effet de surveiller tout à la fois le patient et son ventilateur. En effet, la détection de ce type d'épisodes repose en partie sur l'analyse des tracés de pression et de débit aux voies aériennes fournis par le ventilateur. La multiplicité des évènements, qui de plus peuvent coexister de façon simultanée, rend leur recherche d'autant plus fastidieuse que le soignant ne peut être en permanence au chevet du patient.

D'autre part, quoique présente chez environ un malade sur deux recevant une assistance respiratoire alors qu'il est conscient, l'inconfort respiratoire, ou dyspnée, n'est que très rarement recherché au cours de l'examen du patient sous ventilation mécanique. L'expression verbale est difficile. L'expression non verbale (analyse des mimiques faciales) est probablement utile mais sa valeur clinique n'est pas documentée.

Ainsi, les outils diagnostiques cliniques ne sont pas performants pour détecter une dysharmonie entre le patient et son ventilateur. Ils ne sont par ailleurs pas envisageables chez les patients ventilés à domicile en raison d'une pathologie chronique (20% des patients ventilés le sont en dehors d'une structure de soins), faute de soignant en permanence sur place. Eu égard à la sévérité des complications associées à une dysharmonie patient ventilateur, il

apparaît nécessaire de disposer d'outils permettant de détecter une telle situation, dans le but d'alerter les soignants afin qu'ils y remédient.

Deux des inventeurs ont développé des systèmes de détection de la dysharmonie basés sur l'analyse du signal électro-encéphalographique lié à la ventilation (activité électrique cérébrale recueillie au moyen d'électrodes de surface).

Ainsi le document FR-A-2 903 314 décrit un dispositif et un procédé de détection d'une dysharmonie entre un patient et une machine d'assistance respiratoire consistant, pour chaque cycle respiratoire, à mesurer un signal électro-encéphalographique sur un intervalle de mesure s'étendant autour du début de l'inspiration, puis à moyenner les signaux électro-encéphalographiques mesurés sur plusieurs intervalles de mesure et enfin de traiter le signal moyenné ainsi obtenu pour en déduire une éventuelle dysharmonie entre le patient et la machine d'assistance respiratoire. Un tel procédé ne donne pas entière satisfaction. En effet, les situations d'inconfort ou de détresse respiratoire sont fréquemment associées à des mouvements du patient à l'origine de parasites électriques de l'électroencéphalogramme rendant difficile son interprétation. D'autre part, le procédé de détection décrit dans ce document nécessite de mesurer et de moyenner un signal électro-encéphalographique sur au moins soixante à quatre vingt cycles respiratoires pour être en mesure de conclure à l'existence d'une dysharmonie. Or, soixante à quatre vingt cycles respiratoires correspondent, en moyenne, à quatre à cinq minutes, pendant lesquelles, si une dysharmonie existe, le patient est en détresse. Dans les situations d'inconfort ou de détresse respiratoire résultant d'une dysharmonie entre le patient et le ventilateur, il est important d'être en mesure de détecter cette dysharmonie et d'y remédier le plus rapidement possible pour rétablir l'harmonie entre le patient et son ventilateur.

Afin de s'affranchir de la problématique de la contamination du signal électro-encéphalographique lié à la ventilation et de permettre son traitement plus rapide, le document FR-A-2 962 322 décrit de nouveaux dispositif et procédé d'analyse de l'électroencéphalogramme. Le procédé consiste en un recueil de l'électroencéphalogramme par de multiples électrodes placées sur le scalp du patient. Le signal recueilli est secondairement filtré dans une bande de fréquence comprise entre 0,03 et 40 Hz, et sa puissance calculée. La comparaison de la puissance du signal précédant l'inspiration à celle d'une période de référence permet de mettre en évidence un épisode de dysharmonie lorsque le système objective une diminution de puissance. Cependant, le procédé décrit dans ce document ne donne toutefois pas entière satisfaction. En effet, il ne permet l'analyse que de la partie inspiratoire de la ventilation, excluant la phase expiratoire, alors même que cette dernière peut être source de dysharmonie. Par ailleurs, elle requiert un enregistrement simultané de la ventilation afin de synchroniser l'analyse de l'électroencéphalogramme.

## OBJET DE L'INVENTION

Un but de l'invention est de proposer un procédé de caractérisation de l'état physiologique d'un patient à partir de l'analyse de son activité cérébrale et un dispositif faisant application, en vue de détecter des états spécifiques de telle ou telle situation du patient, et en particulier une situation de dysharmonie avec le dispositif d'assistance médicale auquel le patient est relié.

## RESUME DE L'INVENTION

En vue de la réalisation de ce but, on propose un procédé de détection d'un état physiologique d'un patient déviant d'un état physiologique de référence, dans lequel, après avoir déterminé dans chacune de Q bandes de fréquence un pôle de référence $PR_q$ avec $q \in [1...Q]$ correspondant à l'état physiologique de référence, les étapes suivantes sont répétées en boucle :

- effectuer dans M segments temporels des mesures d'un signal électro-encéphalographique du patient selon n voies et à p instants pour générer M matrices de mesure $X_m$ contenant $n \times p$ échantillons, pour $m \in [1...M]$ ;
- filtrer et centrer chaque matrice de mesure Xm dans les Q bandes de fréquence pour obtenir $M \times Q$ matrices de mesure filtrées $X_{m,q}$ et déterminer $M \times Q$ matrices normées de covariance spatiale $\boldsymbol{C_{m,q}}$ par la formule

$$C_{m,q} = \left(X_{m,q} X_{m,q}^{T}\right) / trace\left(X_{m,q} X_{m,q}^{T}\right);$$

- pour chaque segment temporel m, déterminer des distances $\boldsymbol{d_{m,q}}$ entre chaque matrice normée de covariance spatiale $\boldsymbol{C_{m,q}}$ et le pôle de référence $\boldsymbol{PR_q}$, et déterminer un écart $\boldsymbol{e_m}$ à l'état physiologique de référence en fonction des distances $\boldsymbol{d_{m,q}}$.
- comparer chacun des écarts $\boldsymbol{e_m}$ à l'état physiologique de référence à un seuil S déterminé.

Si le seuil S est dépassé par l'un des écarts, l'état physiologique du patient est alors déclaré déviant par rapport à l'état physiologique de référence.

Les pôles de référence $\boldsymbol{PR_q}$ peuvent être déterminés de plusieurs façons. Selon un premier mode de mise en oeuvre, les pôles de référence $\boldsymbol{PR_q}$ sont constitués par des prototypes ou matrices de référence $\boldsymbol{PR_{q,r}}$ déterminés selon les étapes suivantes :

- effectuer dans M segments temporels des mesures d'un signal électro-encéphalographique du patient selon n voies et à p instants pour générer M matrices de mesure $X_m^{ref}$ de dimension n×p pour m∈ [1...M], alors que le patient est dans l'état physiologique de référence ;
- filtrer et centrer chaque matrice de mesure $X_m^{ref}$ dans les Q bandes de fréquence pour obtenir M×Q matrices de mesure filtrées $\bar{X}_{m,q}^{ref}$ et déterminer M×Q matrices normées de covariance spatiale par la formule

$$C_{m,q}^{ref} = \left(X_{m,q}^{ref} \cdot X_{m,q}^{ref\ T}\right) / trace\left(X_{m,q}^{ref} \cdot X_{m,q}^{ref\ T}\right);$$

- déterminer dans chaque bande de fréquences R prototypes $\boldsymbol{PR_{q,r}}$ r∈ [1...R] à partir des matrices normées de covariance spatiale $C_{m,q}^{ref}$ en utilisant l'algorithme des nuées dynamiques.

Ainsi, le pôle de référence $\boldsymbol{PR_q}$ de chaque bande de fréquence est constitué de l'ensemble des prototypes $\boldsymbol{PR_{q,r}}$ r∈ [1...R] de la bande de fréquence correspondante. Les distances $\boldsymbol{d_{m,q}}$ de chaque matrice normée de covariance spatiale $\boldsymbol{C_{m,q}}$ au pôle de référence sont alors données par $d_{m,q} = \arg\left\{\min_{r=1...R} dist\left(C_{m,q}, PR_{q,r}\right)\right\}$, et l'écart à la situation de référence est donné par $e_m = \sum_{q=1}^{Q} d_{m,q}$

Le seuil S est de préférence déterminé selon les étapes suivantes :

- calculer des distances $d_{m,q}^{ref}$ entre chaque matrice de covariance $C_{m,q}^{ref}$ et celui des prototypes $PR_{q,r}$ qui en est le plus proche ;
- déterminer la distance médiane DM de toutes ces distances $d_{m,q}^{ref}$ ainsi calculées, le seuil S étant déterminé à partir de la moyenne arithmétique MVA des valeurs absolues des écarts de chacune des distances $d_{m,q}^{ref}$ à la distance médiane DM.

Selon un autre mode de mise en oeuvre, le pôle de référence $\boldsymbol{PR_q}$ de chaque bande de fréquence est constitué par la projection sur les courbes principales géodésiques définies par les matrices de covariance $C_{m,q}^{ref}$ qui maximise la quantité d'information (par exemple à au moins 95%). Cette technique est une extension de l'analyse en composantes principales dans le cas des espaces vectoriels (voir Fletcher (2004)).

Les distances $\boldsymbol{d_{m,q}}$ entre chaque matrice normée de covariance spatiale $\boldsymbol{C_{m,q}}$ au pôle de référence correspondant sont alors données par la projection de chacune des matrices normées de covariance spatiales $\boldsymbol{C_{m,q}}$ sur les courbes principales géodésiques correspondantes. L'écart à la situation de référence est donné par $e_{=} = \sum_{q=1}^{Q} d_{m,q}$

De préférence, la distance entre les matrices utilisée dans le procédé de l'invention est la distance riemanienne suivante :

$$dist(P_1,P_2) = \left\{ \sum_{k=1}^{K} \ln^2(\lambda_k) \right\}^{1/2}$$

où les $\lambda_k$ sont les K valeurs propres de la matrice conjointe $P_1^{-1} \cdot P_2$.

Ainsi contrairement aux procédés classiques dans lesquels les mesures électro-encéphalographiques sont exploitées soit par l'étude des potentiels évoqués liés à une référence temporelle (le signal de débit dans ce cas, comme dans FR-A-2 903 314), soit par l'analyse du spectre fréquentiel (comme dans FR-A-2 962 322), le procédé de l'invention privilégie l'étude des relations temporelles entre les composantes phasiques des différents signaux électro-encéphalographiques. L'utilisation de la méthode des synchronies permet de décrire la distribution spatio-temporelle des sources neuronales actives au cours de l'enregistrement électro-encéphalographique, et constitue donc une approche nouvelle des interactions et de la caractérisation du réseau neuronal.

L'invention est également relative à un dispositif de surveillance d'un patient pour détecter un état physiologique déviant d'un état de référence du patient, le dispositif de surveillance comportant des moyens de mesure pour mesurer les signaux électro-encéphalographiques du patient, des moyens de traitement du signal en temps réel pour mettre en oeuvre le procédé de l'invention à partir des signaux ainsi mesurés, et des moyens de réaction pour réagir à un dépassement du seuil.

Selon une première variante, les moyens de réaction comprennent un avertisseur adapté à délivré un signal d'avertissement en réponse au dépassement du seuil.

Selon une deuxième variante, les moyens de réaction comprennent un générateur de consigne à destination d'un dispositif d'assistance médicale auquel le patient est relié, le générateur de consigne étant adapté à faire varier la consigne en réponse au dépassement du seuil pour modifier le fonctionnement du dispositif d'assistance médicale de sorte à faire revenir le patient vers l'état physiologique de référence.

## BREVE DESCRIPTION DES DESSINS

L'invention sera mieux comprise à la lumière de la description qui suit d'un mode de mise en oeuvre particulier, en référence aux figures des dessins annexés, parmi lesquelles :

- la figure 1 est une vue schématique d'un poste médicalisé avec un lit recevant un patient, un dispositif d'assistance respiratoire et un dispositif de surveillance selon l'invention utilisé pour contrôler le dispositif d'assistance respiratoire ;
- la figure 2 est un organigramme décrivant la mise en oeuvre du procédé de l'invention par le dispositif de surveillance de la figure 1 ;
- la figure 3 est un organigramme décrivant l'étape préalable de détermination des matrices de références et du seuil caractérisant l'état physiologique de référence ;
- la figure 4 est un graphe illustrant la mise en oeuvre du procédé de l'invention et la détection d'un état physiologique déviant.

## DESCRIPTION DETAILLEE DE L'INVENTION

L'invention va maintenant être détaillée en référence à son application à la détection de dysharmonie entre un patient et le dispositif d'assistance respiratoire auquel il est relié. Il est ici fait l'hypothèse qu'en cas de dysharmonie, l'activité cérébrale du patient se modifie par rapport à un état physiologique de référence lors duquel le patient est en harmonie avec le dispositif d'assistance respiratoire.

Tout acte cognitif résulte d'une coopération entre plusieurs réseaux neuronaux spatialement distribués (Varela et al, 2001). A l'heure actuelle, et malgré leurs progrès récents, les principales techniques d'imagerie cérébrale (l'électro-encéphalographie, la magnétoencéphalographie, l'imagerie par résonnance magnétique fonctionnelle et la tomographie par émission de positons) ne fournissent qu'une cartographie des activations cérébrales, sans rendre directement compte des interactions entre ces activations.

L'invention repose sur l'hypothèse selon laquelle les liens dynamiques entre les groupes neuronaux se manifestent par la synchronisation d'activités oscillatoires dans une bande de fréquence (Varela et al., 2001). De nombreux résultats expérimentaux chez l'animal obtenus par enregistrements par micro électrodes valident déjà cette hypothèse de synchronie. Chez l'homme, des études ont montré l'existence de synchronisations entre régions distantes, liées au contexte cognitif (Rodriguez et al. 1999).

L'hypothèse d'un rôle des synchronies dans l'intégration des activités cérébrales fut proposée à l'origine par Milner en 1974 pour résoudre le problème de la segmentation figure/fond dans une scène visuelle. Milner proposait que

les neurones répondant à la figure déchargent de façon synchrone, tandis que ceux codant pour le fond déchargent de façon aléatoire. Cette hypothèse fut par la suite reprise par Freeman (Freeman, 1975) et appuyée par des travaux sur le bulbe olfactif du lapin, puis par Von der Marlsburg (Von der Marlsburg, 1981) avant de tomber dans un relatif oubli. Mais la découverte d'oscillations synchrones chez l'animal dans la bande γ (30-70 Hz) par Gray et Singer (Gray et Singer, 1989) dans le cortex visuel du chat relança l'intérêt pour cette idée : cette étude montra que deux neurones du cortex visuel déchargent en phase à des fréquences voisines de 40 Hz en réponse à des stimulations qui semblent provenir du même objet. A l'inverse, si les stimulus ne semblent pas provenir du même objet, les neurones déchargent également, mais pas en synchronie.

Ces résultats s'insèrent dans une approche qui peut s'exprimer en termes d'assemblées cellulaires résonantes (Varela 1995 ; Damasio 1990 ; Llinas, Ribary et al. 1994) : l'émergence de tout acte cognitif correspondrait à la sélection transitoire d'un sous-ensemble distribué de neurones reliés par de fortes connexions réciproques (une assemblée cellulaire, "*cell assembly*"). En raison de son réseau d'interconnexions très denses, le cerveau contient une quasi-infinité d'assemblées de ce type et chaque neurone peut appartenir, à différents instants, à une myriade d'assemblées. La sélection s'opérerait par la mise en synchronie rapide dans une bande de fréquence spécifique des différents neurones appartenant à l'assemblée (la résonance). La synchronie agirait comme une « colle » permettant l'assemblage temporaire de neurones en une assemblée résonante, et par-là même l'intégration neuronale nécessaire à un acte cognitif.

Il est utile de distinguer deux niveaux d'assemblées. A un niveau local, la formation de micro-assemblées au sein d'une même aire corticale permettrait l'intégration d'informations de même nature (visuelles, auditives). Ces liens locaux correspondraient aux synchronies observées chez l'animal entre neurones proches (Singer 1995). Ces micro-assemblées pourraient à leur tour entrer en synchronie pour former des macro-assemblées reliant des zones cérébrales distantes. Ces macro-assemblées permettraient l'intégration de processus de natures différentes au sein d'actes cognitifs complexes.

Le but de la présente invention est d'exploiter ces hypothèses pour reconnaître un changement de l'activité cérébrale lié à une situation inhabituelle par exemple une situation de respiration "modifiée" (contrainte inspiratoire, comme on peut la simuler au laboratoire, ou la rencontrer en pathologie ou sous assistance respiratoire mécanique), par rapport à une activité ou un état physiologique de référence. La reconnaissance d'une telle situation permet par exemple de commander une modification du fonctionnement du dispositif d'assistance respiratoire pour ramener le patient à une situation de confort caractérisée par une activité cérébrale normale.

Le contexte général de l'invention étant posé, on va maintenant détailler le procédé de l'invention. Le principe de l'invention est tout d'abord exposé en référence à la figure 1, sur laquelle on distingue le patient 1 allongé sur un lit 2 et équipé d'un masque 3 relié par une arrivée d'air à un dispositif d'assistance respiratoire 4. Le patient est équipé d'un casque 5 portant n électrodes adaptées à mesurer des signaux électro-encéphalographiques. Le casque 5 est relié à un dispositif de surveillance 6, ici un ordinateur comportant une unité centrale 7 exécutant un programme de surveillance, qui reçoit les mesures électro-encéphalographiques, les traite, et génère en réponse des consignes à destination du dispositif d'assistance respiratoire auquel le dispositif de surveillance 6 est relié.

Le dispositif de surveillance met en oeuvre un algorithme de surveillance en temps réel, illustré à la figure 2, qui met en oeuvre le procédé de l'invention pour surveiller en permanence l'activité électro-encéphalographique du patient. L'avantage du procédé de l'invention est que le cycle de surveillance est très court et le dispositif de surveillance peut ainsi réagir rapidement à la détection d'un état physiologique déviant.

Le dispositif comporte ici des moyens pour réagir à la détection d'un dépassement du seuil, par exemple un haut-parleur 8 pour émettre une alarme et ainsi avertir le personnel soignant, mais est également programmé pour générer une consigne à destination du dispositif d'assistance respiratoire 4 auquel il est relié par un câble 9.

Pour fonctionner, le dispositif de surveillance 6 doit avoir au préalable caractérisé un état physiologique de référence du patient, par exemple un état de confort dans lequel le patient ne ressent aucune gêne respiratoire. Pour ce faire, comme indiqué à la figure 3, le dispositif de surveillance 6 mesure des signaux électro-encéphalographiques du patient en temps réel et effectue ainsi des mesures dans M segments temporels.

Chaque mesure donne lieu à une matrice $X_m^{ref}$ de n signaux mesurés à p instants pendant le segment temporel m, formant ainsi p échantillons dans chaque segment temporel. Puis le dispositif de surveillance 6 filtre et centre chaque matrice de mesure $X_m^{ref}$ dans Q bandes de fréquence (de préférence les cinq bandes de fréquence habituelles pour l'étude de l'électro-encéphalogramme : 1-4 Hz, 4-8 Hz, 8-12 Hz, 12-24 Hz et 24-48 Hz) pour obtenir MxQ matrices de mesure de référence filtrées $X_{m,q}^{ref}$ .

A partir des matrices de mesure filtrées, le dispositif de surveillance 6 détermine MxQ matrices normées de covariance spatiale de référence $C_{m,q}^{ref}$ par la formule :

$$C_{m,q}^{ref} = \left(X_{m,q}^{ref} \cdot X_{m,q}^{ref\ T}\right) / trace\left(X_{m,q}^{ref} \cdot X_{m,q}^{ref\ T}\right).$$

Les matrices de covariance spatiale $C_{m,q}^{ref}$ caractérisent la synchronisation des activités neuronales au cours du temps. Ces matrices de covariance spatiale comportent, dans la diagonale, les synchronisations locales et, hors diagonale, les synchronisations à distance qui sont alors les caractéristiques de la dynamique du réseau neuronal.

Puis, dans chaque bande de fréquence, des matrices de référence ou prototypes $\boldsymbol{PR_{q,r}}$ r∈[1...R] sont déterminées à partir de la répartition des matrices de covariance spatiale $C_{m,q}^{ref}$. Chaque prototype est un représentant d'une sous-classe de la synchronisation, et est ici estimé par une moyenne de Karcher des matrices de Covariance spatiale de référence $C_{m,q}^{ref}$ de voisinage. La procédure de calcul des prototypes $\boldsymbol{PR_{q,r}}$ utilisée dans le présent mode de mise en oeuvre est décrite ci-dessous et est appliquée pour chaque bande de fréquence. Ce calcul repose ici sur l'algorithme des nuées dynamiques (E. Diday, 1971) adapté à la métrique riemannienne. On notera en effet qu'en traitement du signal, on utilise habituellement la norme classique de Frobenius pour définir des distances entre des matrices de covariance (qui sont par définition des matrices Hermitiennes définies positives). Cette approche suppose un espace vectoriel normé de courbure nulle. Cependant, l'espace des matrices Hermitiennes définies positives ressort plutôt des espaces métriques à courbure négative. L'approche proposée dans le cadre de l'invention utilise de préférence les outils de la géométrie Riemannienne pour manipuler les matrices de covariance. Dans ce cadre, la distance entre deux matrices correspond à la géodésique dans l'espace engendré par leur propriété Hermitienne; et la moyenne des matrices de covariance ne correspond plus à une moyenne arithmétique comme classiquement, mais à une moyenne géométrique.

*1: procédure*
*C est un tableau de M matrices de covariance.*
*L est le nombre de prototypes*
*2: k : =M*
*3: eps : = $10^{-8}$*
*4 : kk: =random (k)*
*5: $PR^j$ : =$C_{kk(j)}$ ; j : =1:L; $PR_new^j$ :=0*
*6: tant que abs ($PR_new^j$ - $PR^j$) > eps*
*7: $PR_new^j$ : = $PR^{j;}$*
*8: pour i:=1 à k*
*9: label (i) := arg min j=1;L [**dist** ($PR^j$ , $C^i$)]*
*10: fin pour*
*11: calcul de la moyenne de Karcher pour cha que partition PR*
*12: pour j:=1 à L;*
*13: kj= [label (1 : k) . j] ; $PR^j$ := $\boldsymbol{M_R}$ ($C^{kj}$)*
*14: fin pour*
*15: fin tant que*
*16: retourner PR*

Ici, et selon un aspect particulier de l'invention, la distance ***dist*** entre les matrices utilisée ici est la distance riemanienne suivante : si $\boldsymbol{P_1}$,$\boldsymbol{P_2}$ sont deux matrices, alors

$$dist(P_1, P_2) = \left\{ \sum_{k=1}^{K} \ln^2(\lambda_k) \right\}^{1/2}$$

où les $\lambda_k$ sont les K valeurs propres de la matrice conjointe $\boldsymbol{P_1^{-1} \cdot P_2}$. Cette distance Riemannienne vérifie les trois propriétés d'une distance (symétrie, séparation et inégalité triangulaire).

Le calcul de la moyenne de Karcher $\mathbf{M_R}$ d'un ensemble de matrices peut se réaliser à l'aide d'une procédure de descente de gradient qui converge rapidement (Pennec et al., 2006) :

*1: procédure*
*C est un tableau de M matrices de covariance.*

*2: CM := C(1); d=0 ;*
*3: on initialise la moyenne avec la première valeur du tableau.*
*4: e := $10^{-8}$;*
*5: tant que d > e*
*6:* W *:= 0 ;*
*7: pour i = 1 à M*
*8: W := W +* ***log*** $_{CM}$ *(C(i)) ;*
*9: on somme les vecteurs tangents dans l'espace tangent*
*10: fin pour*
*11: W := W/M;*
*12: on revient sur la variété avec la carte exponentielle et on réitère*
*13: CM_new :=* ***exp*** $_{CM}$ *(W) : distance entre 2 itérations successives*
*14 : d =* ***dist*** *(CM_new, CM)*
*15: CM=CM_new*
*16: fin tan t que*
*17:* $\boldsymbol{M_R}$ *= CM*
*18: retourner* $\boldsymbol{M_R}$*;*

Avec comme opérateurs

$$\mathbf{exp}_{CM}(W) = CM^{½} \exp(CM^{-½}\, W\, CM^{-½})\, CM^{½}$$

$$\mathbf{log}_{CM}(C) = CM^{½} \log(CM^{-½}\, C\, CM^{-½})\, CM^{½}$$

Une fois les matrices de référence ou prototypes $\boldsymbol{PR_{q,r}}$ déterminés, le dispositif de surveillance calcule un seuil S par la méthode suivante. On calcule les distances entre chaque matrice de covariance spatiale de référence $C^{ref}_{m,q}$ et le prototype $\boldsymbol{PR_{q,r}}$ le plus proche, et on détermine la distance médiane DM de toutes les distances ainsi calculées. Le seuil S est alors pris égal à la moyenne arithmétique des valeurs absolues MVA des écarts de chacune des distances ainsi calculées à la distance médiane DM :

*1: procédure de calcul du seuil*
*C est un tableau de M matrices de covariance.*
*L est le nombre de prototypes*
*2: pour i:=1 à M*
*3: d(i) := arg min j=1:L [***dist*** *($S^j$ , $C^i$)]) ;*
*4: fin pour*
*5: MVA := moyenne arithmétique(valeur absolue(dist-médiane(dist)))*
*6:* $S := MVA + \sqrt{4 \log(\text{nombre voie EEG})}\ \times MVA$
*7: return S*

En variante, le seuil peut être déterminé de tout autre façon. Par exemple, on peut retenir comme seuil la moyenne statistique de toutes les distances ainsi calculées, augmentée de trois écarts-types.

L'étape préalable de caractérisation de l'état physiologique de référence étant maintenant réalisé, le dispositif de surveillance 6 peut mettre en oeuvre une surveillance de l'état physiologique du patient en temps réel comme suit.

Comme indiqué à la figure 2, le dispositif de surveillance 6 mesure des signaux électro-encéphalographiques du patient en temps réel et effectue ainsi des mesures dans M segments temporels sont ainsi effectuées.

Chaque mesure donne lieu à une matrice $\boldsymbol{X_m}$ de n signaux mesurés à p instants pendant le segment temporel m. Puis le dispositif de surveillance 6 filtre et centre chaque matrice de mesure $\boldsymbol{X_m}$ dans Q bandes de fréquence (de préférence les cinq bandes de fréquence habituelles 1-4 Hz, 4-8 Hz, 8-12 Hz, 12-24 Hz et 24-48 Hz) pour obtenir MxQ matrices de mesure filtrées $\boldsymbol{X_{m,q}}$.

A partir des matrices de mesure filtrées, on détermine MxQ matrices normées de covariance spatiale $\boldsymbol{C_{m,q}}$ par la formule

$$C_{m,q} = \left(X_{m,q} X_{m,q}^{T}\right) / trace\left(X_{m,q} X_{m,q}^{T}\right).$$

Pour chaque segment temporel m, le dispositif de surveillance calcul un écart à l'état physiologique de référence $\mathbf{e_m}$ par :

$$e_m = \sum_{q=1}^{Q} \arg\left\{\min_{r=1...R} dist\left(C_{m,q}, PR_{q,r}\right)\right\}.$$

Si l'un des écarts ainsi déterminés est plus grand que le seuil S, c'est alors le signe que l'état physiologique du patient est éloigné de l'état physiologique de référence.

Dans ce cas, le dispositif de surveillance 6 modifie la consigne envoyée au dispositif d'assistance respiratoire 4 pour en modifier le fonctionnement dans un sens tendant à ramener le patient vers l'état physiologique de référence.

Les étapes de la figure 2 sont répétées en boucle pour permettre une surveillance permanente du patient.

Les p échantillons temporels des n signaux constituant les matrices de mesure $\boldsymbol{X_m}$ ou $X_m^{ref}$ peuvent être obtenus en utilisant n voies électro-encéphalographiques distinctes, ce qui permet de prendre en compte l'extension spatiale de l'activité encéphalographique. Cependant, il est possible de reconstruire n signaux utilisables à partir d'un nombre r plus restreint de voies électro-encéphalographiques par la technique du plongement temporel (voir Lachaux & al, 1997). Pour cela, il suffit de déterminer pour chaque voie électro-encéphalographique un délai temporel ΔT qui est de préférence pris égal au délai temporel pris par le retour à la valeur 1/e de la fonction d'autocorrélation de la voie électro-encéphalographique concernée. D'une voie électro-encéphalographique donnée V, on peut ainsi reconstruire q voies virtuelles, donnant autant de signaux utilisables :

V(t1), V(t1+ ΔT)..., V(t1+k ΔT)

V(t2), V(t2+ ΔT)..., V(t2+k ΔT)

...

V(tq), V(tq+ ΔT)..., V(tq+k ΔT)

Le paramètre k est appelé plongement temporel. A l'extrême, il est possible de n'utiliser qu'une seule voie électro-encéphalographique pour reconstruire n voies virtuelles donnant les signaux utilisables dans le cadre de l'invention. On peut également mixer les deux méthodes pour obtenir un plongement spatio-temporel, en utilisant r voies électro-encéphalographiques de chacune desquelles on reconstruit q voies virtuelles de sorte que rxq=n.

Le graphique de la figure 4 montre un essai d'identification selon le procédé de l'invention de l'état physiologique d'un patient induit par des modifications encéphalographiques consécutives à une contrainte respiratoire.

La partie de la courbe référencée VS correspond à une situation de ventilation spontanée utilisé comme référence, tandis que la partie de la courbe intitulée charge correspond à une dysharmonie provoquée. Le graphe du haut est l'écart entre l'état de référence et l'état physiologique actuel du patient, tracée en fonction du temps.

Le graphe du bas est le résultat de la détection de l'état dysharmonique, pour différentes périodes d'intégration sur des fenêtres mobiles de 4, 8, 12 secondes, respectivement. On constate que quelques fausses alarmes sont détectées en situation de ventilation spontanée sur 500 fenêtres analysées (0.6 %) suivant l'intégration réalisée sur une durée totale de 2000 secondes. On constate que la situation de dysharmonie est détectée à coup sûr par la mise en oeuvre du procédé de l'invention.

L'invention n'est pas limitée à ce qui vient d'être décrit, mais englobe au contraire toute variante entrant dans le cadre défini par les revendications.

En particulier, bien que le dispositif de surveillance est ici extérieur au dispositif d'assistance, il peut bien sûr être intégré dans ce dernier.

REFERENCES

DAMASIO, A.R. (1990). " Synchronous activation in multiple cortical régions : a mechanism for recall." Semin. Neurosci., 2: 287-297.

DIDAY E. (1971) - « Une nouvelle méthode en classification automatique et reconnaissance des formes : la méthode des nuées dynamiques », Revue de Statistique Appliquée, XIX (2), pp.19-33.

FREEMAN, W.J. (1975). "Mass Action in the nervous system." Academic Press, New York.

Fletcher T., Conglin Lu, Stephen M. Pizer, and Sarang C. Joshi. (2004). "Principal geodesic analysis for the study of nonlinear statistics of shape." IEEE Transactions on Medical Imaging, 23(8):995, 2004.

GRAY, C.M., and W. SINGER. (1989). "Stimulus-specific neuronal oscillations in orientation columns of cat visual cortex." Proc. Natl. Acad. Sci. USA, 86 (5): 1698-1702.

Lachaux J.-P., Pezard L. Garnero L. Pelte C. Renault B. Varela F. J., Martinerie J. "Spatial Extension of Brain Activity Fools the Single-Channel Reconstruction of EEG Dynamics.", Human Brain Mapping 5:26-47 (1997)

LLINAS, R., U. RIBARY, et al. (1994). " Content and context in temporal thalamocortical binding." In : Buzsaki G. (ed) : Temporal coding in the brain. Berlin and Heidelberg. Springer-Verlag., 251-272.

MILNER, P.M. (1974). " A model for visual shape recognition." Psychol Rev., 81: 521-535.

MOAKHER M. (2005) "A differential geometric approach to the geometric mean of symmetric Positive-Definite matrices". SIAM J. Matrix Anal. Appl., 26 : 735-747.

PENNEC X., FILLARD P., and AYACHE N. (2006) "A Riemannian Framework for Tensor Computing," Int'l J. Computer Vision, 66: 41-66

RODRIGUEZ E., GEORGE N., LACHAUX J.P., MARTINERIE J., RENAULT B. and VARELA F. (1999) Perception shadow: long distance gamma band synchronisation and desynchronization on the human scalp. Nature, 397: 430-433.

RODRIGUEZ E., JERBI K., LACHAUX JP. And MARTINERIE J.(2010) Brainweb 2.0 : the quest for synchrony in Ten years of Nature Reviews Neuroscience: insights from the highly cited, Nature Reviews Neuroscience, 11, pp 718-726 , 201

SINGER, W. (1995). " Time as coding space in neocortical processing : a hypothesis." In : Gazzaniga MS (ed) : The cognitive neurosciences. Cambridge, London : The MIT Press. 91-104.

VARELA, F.J. (1995). " Resonant cell assemblies: a new approach to cognitive function and neuronal synchrony." Biol. Res., 28: 81-95.

VARELA F., LACHAUX J.P., RODRIGUEZ E. & MARTINERIE J. (2001) The Brainweb: Phase synchronization and Large-scale integration. Nature Rev Neurosci., 2: 229-239.

VON DER MARLSBURG, C. (1981). " The correlation theory of brain function." Internal Report, 81- 2. Max-Plank-Institut für Biophysikalische Chemie.

## Revendications

**1.** Procédé de détection d'un état physiologique d'un patient déviant d'un état physiologique de référence, dans lequel, après avoir déterminé dans chacune de Q bandes de fréquence un pôle de référence $\boldsymbol{PR_q}$ avec $q \in [1...Q]$ représentatif de l'état physiologique de référence, les étapes suivantes sont répétées en boucle :

- effectuer dans M segments temporels des mesures d'un signal électro-encéphalographique du patient selon n voies et à p instants pour générer M matrices de mesure $\boldsymbol{X_m}$ comportant nxp échantillons, pour m∈[1...M] ;
- filtrer et centrer chaque matrice de mesure Xm dans les Q bandes de fréquence pour obtenir MxQ matrices de mesure filtrées $X_{m,q}$ et déterminer MxQ matrices normées de covariance spatiale par la formule

$$C_{m,q} = \left(X_{m,q} X_{m,q}{}^{T}\right) / trace\left(X_{m,q} X_{m,q}{}^{T}\right);$$

- pour chaque segment temporel m, déterminer des distances $\boldsymbol{d_{m,q}}$ entre chaque matrice normée de covariance spatiale $\boldsymbol{C_{m,q}}$ et le pôle de référence $\boldsymbol{PR_q}$, et déterminer un écart $\boldsymbol{e_m}$ à l'état physiologique de référence en fonction des distances $\boldsymbol{d_{m,q}}$.
- comparer chacun des écarts $\boldsymbol{e_m}$ à l'état physiologique de référence à un seuil S déterminé.

**2.** Procédé selon la revendication 1, dans lequel l'étape de mesure et les étapes de calcul sont effectuées en temps réel.

**3.** Procédé selon la revendication 1, dans lequel chaque pôle de référence $\boldsymbol{PR_q}$ est constitué de R matrices de référence ou protoypes $\boldsymbol{PR_{q,r}}$ qui sont déterminées de la façon suivante :

- effectuer dans M segments temporels des mesures d'un signal électro-encéphalographique du patient selon n voies et à p instants pour générer M matrices de mesure $X_m^{ref}$ de dimension nxp pour m∈[1...M], alors que le patient est dans l'état physiologique de référence ;
- filtrer et centrer chaque matrice de mesure $X_m^{ref}$ dans les Q bandes de fréquence pour obtenir MxQ matrices de mesure filtrées $X_{m,q}^{ref}$ et déterminer MxQ matrices normées de covariance spatiale par la formule

$$C_{m,q}^{ref} = \left(X_{m,q}^{ref} \cdot X_{m,q}^{ref\ T}\right) / trace\left(X_{m,q}^{ref} \cdot X_{m,q}^{ref\ T}\right);$$

- déterminer dans chaque bande de fréquences les R matrices de référence ou prototypes $\boldsymbol{PR_{q,r}}$ r∈[1...R] à partir des matrices normées de covariance $C_{m,q}^{ref}$ en utilisant l'algorithme des nuées dynamiques.

**4.** Procédé selon la revendication 3, dans lequel le seuil S est déterminé selon les étapes suivantes :

- calculer des distances $d_{m,q}^{ref}$ entre chaque matrice de covariance $C_{m,q}^{ref}$ et celui des prototypes $PR_{q,r}$ qui en est le plus proche ;
- déterminer la distance médiane DM de toutes ces distances $d_{m,q}^{ref}$ calculées, le seuil S étant pris égal à la moyenne arithmétique des valeurs absolues MVA des écarts de chacune des distances $\boldsymbol{d_{m,q}}$ à la distance médiane DM.

**5.** Procédé selon la revendication 3, dans lequel, pour chaque bande de fréquence, les distances $\boldsymbol{d_{m,q}}$ entre chaque matrice normée de covariance et le pole de référence sont déterminées par $d_{m,q} = \arg\left\{\min_{r=1...R} dist\left(C_{m,q}, PR_{q,r}\right)\right\}$.

**6.** Procédé selon la revendication 5, dans lequel L'écart à la situation de référence est donné par la somme des distances $d_{m,q}$ : $e_m = \sum_{q=1}^{Q} d_{m,q}$ .

**7.** Procédé selon la revendication 3, dans lequel le pôle de référence $\boldsymbol{PR_q}$ de chaque bande de fréquence est constitué par la courbe principale géodésique définie par les matrices de covariance $C_{m,q}^{ref}$ qui maximise la quantité d'information, de préférence à au moins 95%.

**8.** Procédé selon les revendications précédentes, dans lequel les distances sont calculées conformément à la distance riemanienne entre deux matrices $\boldsymbol{P_1},\boldsymbol{P_2}$ suivante :

$$dist(P_1, P_2) = \left\{ \sum_{k=1}^{K} \ln^2(\lambda_k) \right\}^{1/2}$$

où les $\lambda_k$ sont les K valeurs propres de la matrice conjointe $\boldsymbol{P_1^{-1} \cdot P_2}$.

**9.** Procédé selon l'une des revendications précédentes, dans lequel au moins certaines des voies utilisées pour la détermination des matrices de mesure sont des voies virtuelles reconstruites à partir d'au moins une voie électro-encéphalographique par la méthode du plongement temporel.

**10.** Dispositif de surveillance pour mettre en oeuvre le procédé selon l'une des revendications précédentes, comportant :

- des moyens de mesure (5) de signaux électro-encéphlographique pour mesurer les signaux électro-encéphalogrpahiques d'un patient ;
- des moyens de traitement de signal en temps réel (7) pour mettre en oeuvre le procédé selon la revendication 1, comprenant au moins des moyens détermination des pôles de référence et du seuil, et des moyens de calcul d'écarts à l'état physiologique de référence ;
- des moyens de réaction (8) pour réagir à un dépassement du seuil.

**11.** Dispositif de surveillance selon la revendication 10, dans lequel les moyens de réaction comprennent des moyens d'avertissement pour avertir du dépassement du seuil.

**12.** Dispositif de surveillance selon la revendication 10, dans lequel les moyens de réaction comprennent un générateur de consigne à destination d'un dispositif d'assistance médicale auquel le patient est relié, le générateur de consigne étant adapté à faire varier la consigne en réponse au dépassement du seuil pour modifier le fonctionnement du dispositif d'assistance médicale de sorte à faire revenir le patient vers l'état physiologique de référence.

**13.** Dispositif d'assistance médicale comportant un dispositif de surveillance selon la revendication 10.

## Patentansprüche

**1.** Verfahren zur Feststellung eines von einem physiologischen Referenzzustand abweichenden physiologischen Zustands eines Patienten, wobei nach Bestimmung eines Referenzpols *PRq* mit q$\in$ [1...Q], welcher repräsentativ für den physiologischen Referenzzustand ist, in jedem der Q-Frequenzbänder, die folgenden Schritte in einer Schleife wiederholt werden:

- Durchführen von Messungen eines elektroenzephalographischen Signals des Patienten in M-Zeitabschnitten entlang n-Kanälen und zu p-Zeitpunkten zur Erzeugung von M-Messmatrizen $X_m$, welche nxp-Muster umfassen, für m$\in$ [1...M];
- Filtern und Zentrieren jeder der Messmatrizen $X_m$ in den Q-Frequenzbändern zum Erhalten von gefilterten MxQ-Messmatrizen $X_{m,q}$ und Bestimmen von normierten MxQ-Matrizen mit räumlicher Kovarianz durch die Formel

$$C_{m,q} = \left(X_{m,q} X_{m,q}^{\ T}\right) / trace\left(X_{m,q} X_{m,q}^{\ T}\right);$$

- Bestimmen der Distanzen $d_{m,q}$ zwischen jeder normierten Matrix mit räumlicher Kovarianz $C_{m,q}$ für jeden m-Zeitabschnitt und des Referenzpols $PR_q$, und Bestimmen einer Abweichung $e_m$ von dem physiologischen Referenzzustand anhand der Distanzen $d_{m,q}$,
- Vergleichen jeder der Abweichungen $e_m$ von dem physiologischen Referenzzustand mit einem bestimmten Schwellenwert S.

**2.** Verfahren nach Anspruch 1, wobei der Messschritt und die Berechnungsschritte in Echtzeit durchgeführt werden.

**3.** Verfahren nach Anspruch 1, wobei jeder Referenzpol *PRq* aus R-Referenzmatrizen oder Prototypen $PR_{q,r}$ gebildet ist, welche auf folgende Weise bestimmt werden:

- Durchführen von Messungen eines elektroenzephalographischen Signals eines Patienten in M-Zeitabschnitten entlang n-Kanälen und zu p-Zeitpunkten zur Erzeugung von nxp-dimensionierten M-Messmatrizen $X_m^{ref}$ für m∈ [1...M], während der Patient sich in dem physiologischen Referenzzustand befindet;
- Filtern und Zentrieren jeder der Messmatrizen $X_m^{ref}$ in den Q-Frequenzbändern zum Erhalten von gefilterten MxQ-Messmatrizen $X_m^{ref}$ und Bestimmen von normierten MxQ-Matrizen mit räumlicher Kovarianz durch die Formel

$$C_{m,q}^{ref} = \left(X_{m,q}^{ref} \cdot X_{m,q}^{ref\,T}\right) / trace\left(X_{m,q}^{ref} \cdot X_{m,q}^{ref\,T}\right);$$

- Bestimmen der R-Referenzmatrizen oder Prototypen $\boldsymbol{PR_{q,r}}$ r∈ [1...R] in jedem der Frequenzbänder anhand der normierten Kovarianzmatrizen $C_{m,q}^{ref}$ durch den Algorithmus dynamischer Cluster.

**4.** Verfahren nach Anspruch 3, wobei der Schwellenwert S gemäß den folgenden Schritten bestimmt wird:
- Berechnen der Distanzen $d_{m,q}^{ref}$ zwischen jeder der Kovarianzmatrizen $C_{m,q}^{ref}$ und der ihnen nächstgelegenen Prototypen $\boldsymbol{PR_{q,r}}$**;**
- Bestimmen der mittleren Distanz DM aller dieser berechneten Distanzen $d_{m,q}^{ref}$**,** wobei der Schwellenwert S gleich dem arithmetischen Durchschnitt der absoluten Abweichungswerte MVA jeder Distanz $d_{m,q}$ von der mittleren Distanz DM genommen wird.

**5.** Verfahren nach Anspruch 3, wobei die Distanzen $d_{m,q}$ zwischen jeder normierten Kovarianzmatrix und dem Referenzpol für jedes Frequenzband anhand $d_{m,q} = \arg\left\{\min_{r=1...R} dist\left(C_{m,q}, PR_{q,r}\right)\right\}$ bestimmt werden.

**6.** Verfahren nach Anspruch 5, wobei die Abweichung von der Referenzsituation durch die Summe der Distanzen $d_{m,q} : e_m = \sum_{q=1}^{Q} d_{m,q}$ gegeben wird.

**7.** Verfahren nach Anspruch 3, wobei der Referenzpol ***PRq*** jeden Frequenzbandes aus der von den Kovarianzmatrizen $C_{m,q}^{ref}$ definierten geodätischen Hauptkurve gebildet ist, wodurch die Informationsmenge maximiert wird, vorzugsweise um mindestens 95 %.

**8.** Verfahren nach den vorhergehenden Ansprüchen, wobei die Distanzen entsprechend der folgenden Riemann'schen Distanz zwischen zwei Matrizen $\boldsymbol{P_1},\boldsymbol{P_2}$ berechnet werden:

$$dist\left(P_1, P_2\right) = \left\{\sum_{k=1}^{K} \ln^2\left(\lambda_k\right)\right\}^{1/2}$$

wobei $\lambda_k$ die spezifischen K-Eigenwerte der gemeinsamen Matrix $P_1^{-1} \cdot P_2$ sind.

**9.** Verfahren nach einem der vorhergehenden Ansprüche, wobei mindestens einige der für die Bestimmung der Messmatrizen benutzten Kanäle virtuelle Kanäle sind, welche aus mindestens einem elektroenzephalographischen Kanal

durch das Zeit-Einbettungsverfahren rekonstruiert werden.

**10.** Überwachungsvorrichtung zum Umsetzen des Verfahrens nach einem der vorhergehenden Ansprüche, umfassend:

- elektroenzephalographisches Signal-Messmittel (5) zum Messen der elektroenzephalographischen Signale eines Patienten;
- Echtzeit-Signalverarbeitungsmittel (7) zum Umsetzen des Verfahrens nach Anspruch 1, umfassend wenigstens Mittel zur Bestimmung der Referenzpole und des Schwellenwerts, und Mittel zur Berechnung der Abweichungen von dem physiologischen Referenzzustand;
- Reaktionsmittel (8) zum Reagieren auf eine Schwellenwertüberschreitung.

**11.** Überwachungsvorrichtung nach Anspruch 10, wobei die Reaktionsmittel Warnmittel zum Ankündigen der Schwellenwertüberschreitung umfassen.

**12.** Überwachungsvorrichtung nach Anspruch 10, wobei die Reaktionsmittel
eine Sollwert-Erzeugungsvorrichtung umfassen, welche für eine medizinische Versorgungsvorrichtung vorgesehen ist, an welche der Patient angeschlossen ist, wobei die Sollwert-Erzeugungsvorrichtung daran angepasst ist, den Sollwert als Reaktion auf eine Schwellenwertüberschreitung zu variieren, um die Bedienung der medizinischen Versorgungsvorrichtung zu ändern, so dass sie den Patienten zum physiologischen Referenzzustand zurückführt.

**13.** Medizinische Versorgungsvorrichtung, eine Überwachungsvorrichtung nach Anspruch 10 umfassend.

## Claims

**1.** A method for detecting a physiological state of a patient deviating from a reference physiological state, in which, after having determined, in each of Q frequency bands, a reference pole $\boldsymbol{PR_q}$ with q∈[1...Q] representative of the reference physiological state, the following steps are repeated in a loop:

- performing measurements, in M time segments, of an electro-encephalographic signal of the patient along n pathways and at p instants to generate M measurement matrices $\boldsymbol{X_m}$ comprising nxp samples, for m∈[1...M] ;
- filtering and centering each measurement matrix Xm in the Q frequency bands to obtain MxQ filtered measurement matrices $\boldsymbol{X_{m,q}}$ and determining MxQ standardized matrices of spatial covariance by the formula

$$C_{m,q} = \left(X_{m,q} X_{m,q}{}^{T}\right) / trace\left(X_{m,q} X_{m,q}{}^{T}\right);$$

- for each time segment m, determining distances $\boldsymbol{d_{m,q}}$ between each standardized matrix of spatial covariance $\boldsymbol{C_{m,q}}$ and the reference pole $PR_q$, and determining a deviation $\boldsymbol{e_m}$ from the reference physiological state as a function of the distances $\boldsymbol{d_{m,q}}$;
- comparing each of the deviations $\boldsymbol{e_m}$ from the reference physiological state to a determined threshold S.

**2.** The method as claimed in claim 1, in which the measurement step and the computation steps are performed in real time.

**3.** The method as claimed in claim 1, in which each reference pole $\boldsymbol{PR_q}$ is made up of R reference matrices or prototypes $\boldsymbol{PR_{q,r}}$ which are determined as follows:

- performing measurements, in M time segments, of an electro-encephalographic signal of the patient along n pathways and at p instants to generate M measurement matrices $X_m^{ref}$ of size nxp for m∈[1...M], while the patient is in the reference physiological state;
- filtering and centering each measurement matrix Xm in the Q frequency bands to obtain MxQ filtered measurement matrices $X_{m,q}^{ref}$ and determining MxQ standardized matrices of spatial covariance by the formula

$$C_{m,q}^{ref} = \left(X_{m,q}^{ref} \cdot X_{m,q}^{ref\ T}\right) / trace\left(X_{m,q}^{ref} \cdot X_{m,q}^{ref\ T}\right);$$

- determining, in each frequency band, the R reference matrices or prototypes $PR_{q,r}$ r∈[1...R] from the standardized matrices of covariance $C_{m,q}^{ref}$ by using the dynamic swarms algorithm.

**4.** The method as claimed in claim 3, in which the threshold S is determined according to the following steps:

- computing distances $d_{m,q}^{ref}$ between each covariance matrix $C_{m,q}^{ref}$ and the prototype of the prototypes $PR_{q,r}$ which is the closest thereto;
- determining the median distance DM of all these computed distances $d_{m,q}^{ref}$, the threshold S being taken to be equal to the arithmetic mean of the absolute values MVA of the deviations of each of the distances $d_{m,q}$ from the median distance DM.

**5.** The method as claimed in claim 3, in which, for each frequency band, the distances $d_{m,q}$ between each standardized matrix of covariance and the reference pole are determined by $d_{m,q} = \arg\left\{\min_{r=1...R} dist\left(C_{m,q}, PR_{q,r}\right)\right\}$.

**6.** The method as claimed in claim 5, in which the deviation from the reference situation is given by the sum of the distances $d_{m,q}$ : $e_{=} = \sum_{q=1}^{Q} d_{m,q}$ .

**7.** The method as claimed in claim 3, in which the reference pole $PR_q$ of each frequency band is made up of the main geodesic curve defined by the covariance matrices $C_{m,q}^{ref}$ which maximizes the quantity of information, preferably to at least 95%.

**8.** The method as claimed in the preceding claims, in which the distances are computed in accordance with the Riemannian distance between two following matrices $P_1, P_2$ :

$$dist(P_1, P_2) = \left\{\sum_{k=1}^{K} \ln^2(\lambda_k)\right\}^{1/2}$$

in which the $\lambda_k$ are K specific values of the joint matrix $P_1^{-1} \cdot P_2$.

**9.** The method as claimed in one of the preceding claims, in which at least some of the pathways used for the determination of the measurement matrices are virtual pathways reconstructed from at least one electro-encephalographic pathway by the time plunge method.

**10.** A monitoring device for implementing the method as claimed in one of the preceding claims, comprising:

- electro-encephalographic signal measurement means (5) for measuring the electro-encephalographic signals of a patient;
- real-time signal processing means (7) for implementing the method as claimed in claim 1, comprising at least means for determining reference poles and the threshold, and means for computing deviations from the reference situation;
- reaction means (8) for reacting to a threshold overshoot.

**11.** The monitoring device as claimed in claim 10, in which the reaction means comprise warning means for warning of the threshold overshoot.

**12.** The monitoring device as claimed in claim 10, in which the reaction means comprise a setpoint generator intended

for a medical assistance device to which the patient is connected, the setpoint generator being adapted to vary the setpoint in response to the threshold overshoot to modify the operation of the medical assistance device in such a way as to return the patient to the reference physiological state.

**13.** A medical assistance device comprising a monitoring device as claimed in claim 10.

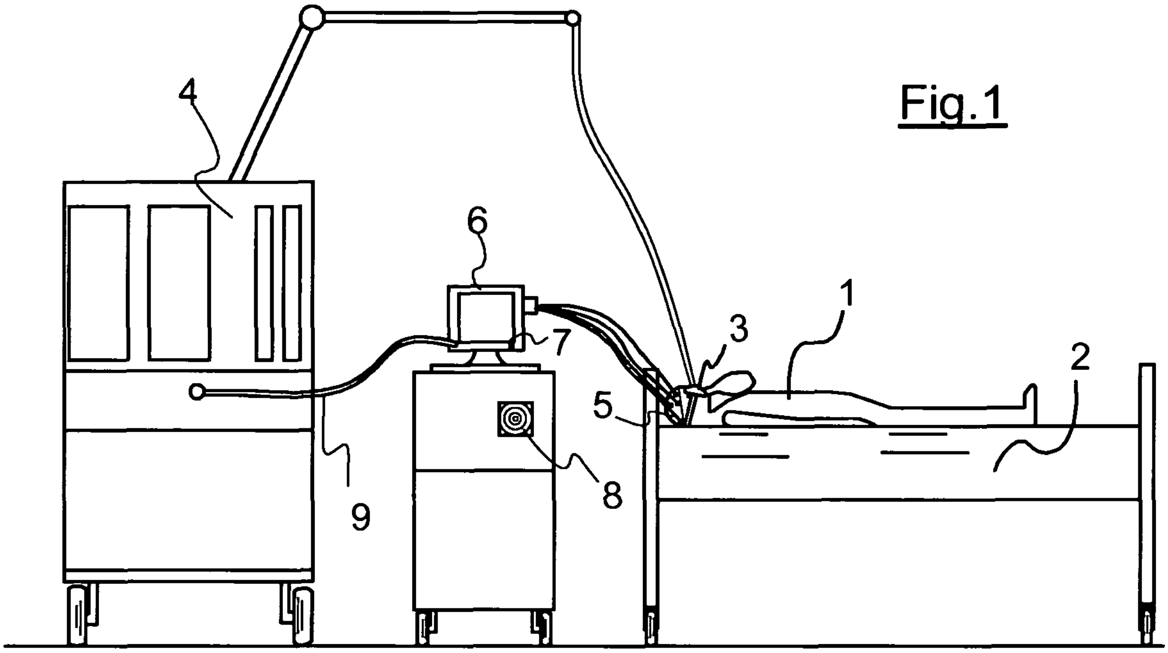
4
6
7
1
3
2
5
8
9

Fig.1

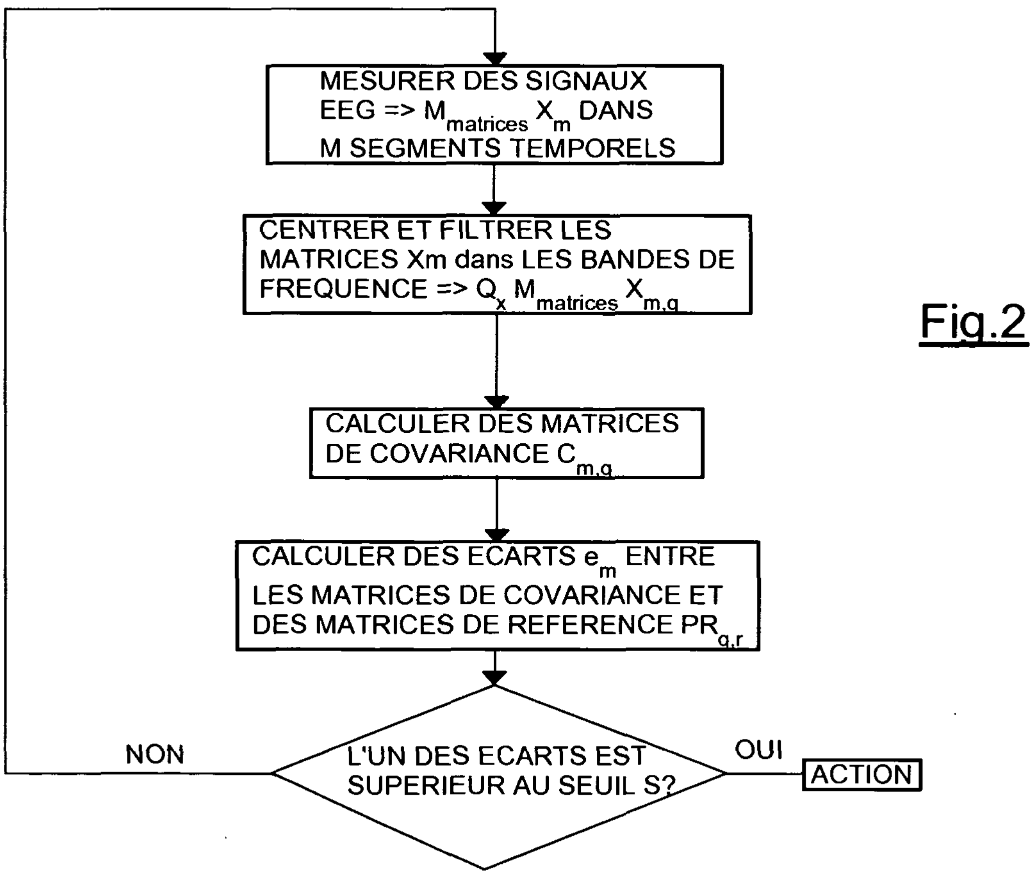
MESURER DES SIGNAUX EEG => $M_{matrices}$ $X_m$ DANS M SEGMENTS TEMPORELS
CENTRER ET FILTRER LES MATRICES Xm dans LES BANDES DE FREQUENCE => $Q_x$ $M_{matrices}$ $X_{m,q}$
CALCULER DES MATRICES DE COVARIANCE $C_{m,q}$
CALCULER DES ECARTS $e_m$ ENTRE LES MATRICES DE COVARIANCE ET DES MATRICES DE REFERENCE $PR_{q,r}$
L'UN DES ECARTS EST SUPERIEUR AU SEUIL S?
NON
OUI
ACTION

Fig.2

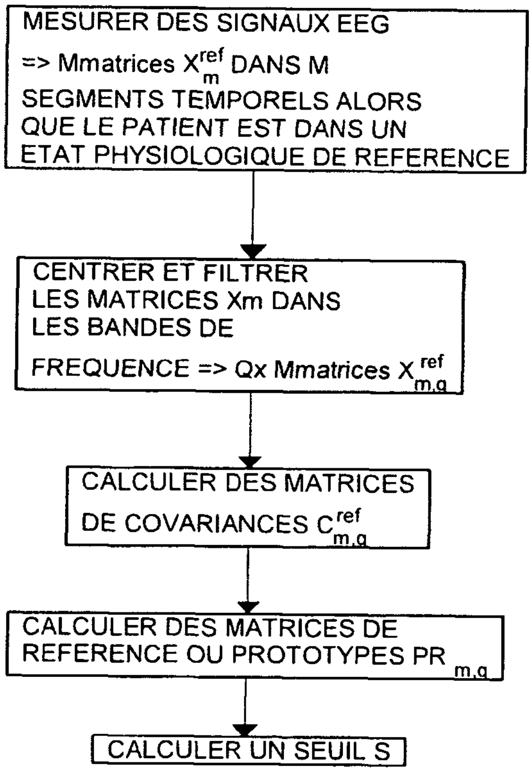


Fig.3

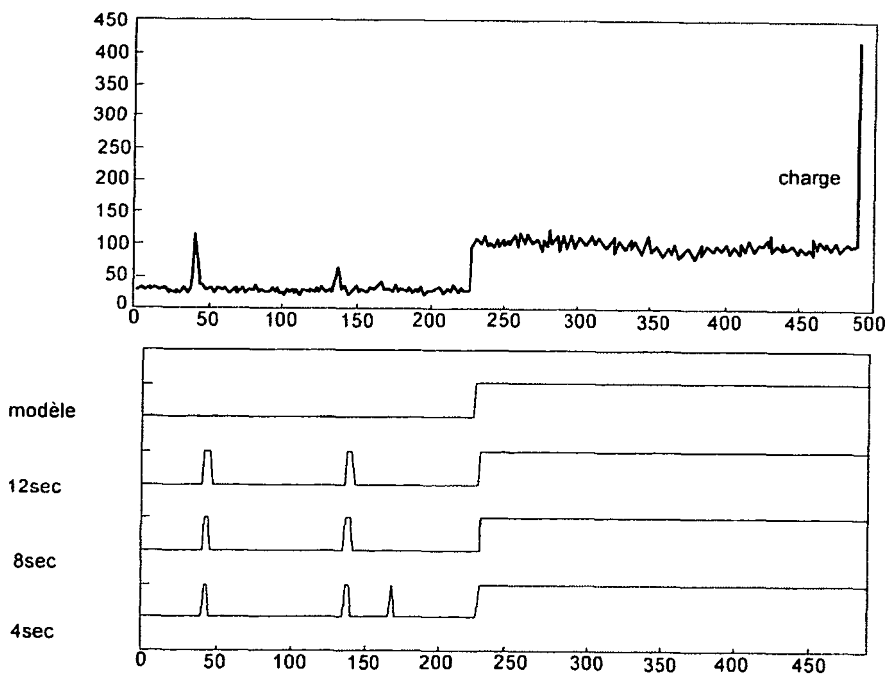


Fig.4

FEUILLE DE REMPLACEMENT (RÈGLE 26)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2903314 A
- FR 2962322 A


### Littérature non-brevet citée dans la description


- **DAMASIO, A.R.** Synchronous activation in multiple cortical régions : a mechanism for recall. *Semin. Neurosci.,* 1990, vol. 2, 287-297
- **DIDAY E.** Une nouvelle méthode en classification automatique et reconnaissance des formes : la méthode des nuées dynamiques. *Revue de Statistique Appliquée,* 1971, vol. XIX (2), 19-33
- **FREEMAN, W.J.** Mass Action in the nervous system. Academic Press, 1975
- **FLETCHER T. ; CONGLIN LU ; STEPHEN M. PIZER ; SARANG C. JOSHI.** Principal geodesic analysis for the study of nonlinear statistics of shape. *IEEE Transactions on Medical Imaging,* 2004, vol. 23 (8), 995
- **GRAY, C.M. ; W. SINGER.** Stimulus-specific neuronal oscillations in orientation columns of cat visual cortex. *Proc. Natl. Acad. Sci. USA,* 1989, vol. 86 (5), 1698-1702
- **LACHAUX J.-P. ; PEZARD L. ; GARNERO L. ; PELTE C. ; RENAULT B. ; VARELA F. J. ; MARTINERIE J.** Spatial Extension of Brain Activity Fools the Single-Channel Reconstruction of EEG Dynamics. *Human Brain Mapping,* 1997, vol. 5, 26-47
- Content and context in temporal thalamocortical binding. **LLINAS, R. ; U. RIBARY et al.** Temporal coding in the brain. Berlin and Heidelberg. Springer-Verlag, 1994, 251-272
- **MILNER, P.M.** A model for visual shape recognition. *Psychol Rev.,* 1974, vol. 81, 521-535
- **MOAKHER M.** A differential geometric approach to the geometric mean of symmetric Positive-Definite matrices. *SIAM J. Matrix Anal. Appl.,* 2005, vol. 26, 735-747
- **PENNEC X. ; FILLARD P. ; AYACHE N.** A Riemannian Framework for Tensor Computing. *Int'l J. Computer Vision,* 2006, vol. 66, 41-66
- **RODRIGUEZ E. ; GEORGE N. ; LACHAUX J.P. ; MARTINERIE J. ; RENAULT B. ; VARELA F.** Perception shadow: long distance gamma band synchronisation and desynchronization on the human scalp. *Nature,* 1999, vol. 397, 430-433
- **RODRIGUEZ E. ; JERBI K. ; LACHAUX JP ; MARTINERIE J.** Brainweb 2.0 : the quest for synchrony in Ten years of Nature Reviews Neuroscience: insights from the highly cited. *Nature Reviews Neuroscience,* 2010, vol. 11, 718-726
- Time as coding space in neocortical processing : a hypothesis. **SINGER, W.** The cognitive neurosciences. The MIT Press, 1995, 91-104
- **VARELA, F.J.** Resonant cell assemblies: a new approach to cognitive function and neuronal synchrony. *Biol. Res.,* 1995, vol. 28, 81-95
- **VARELA F. ; LACHAUX J.P. ; RODRIGUEZ E. ; MARTINERIE J.** The Brainweb: Phase synchronization and Large-scale integration. *Nature Rev Neurosci.,* 2001, vol. 2, 229-239
- The correlation theory of brain function. **VON DER MARLSBURG, C.** Internal Report. Max-Plank-Institut für Biophysikalische Chemie, 1981, vol. 81, 2